(19) Europäisches Patentamt
European Patent Office
Office européen des brevets

(11) Publication number: **0 186 520 A2**

# (12) EUROPEAN PATENT APPLICATION

(21) Application number: 85309514.9

(22) Date of filing: 24.12.85

(51) Int. Cl.[4]: C 12 P 17/06
C 07 D 311/92, A 61 K 35/66
//(C12P17/06, C12R1:365)

(30) Priority: 27.12.84 JP 278633/84

(43) Date of publication of application:
02.07.86 Bulletin 86/27

(84) Designated Contracting States:
DE FR GB

(71) Applicant: YAMANOUCHI PHARMACEUTICAL CO. LTD.
No. 5-1 Nihonbashi-Honcho, 2-chome Chuo-ku
Tokyo(JP)

(72) Inventor: Imai, Harumitsu
No.599, Nakahara-ku Kizuki
Kawasaki-shi Kanagawa(JP)

(72) Inventor: Suzuki, Ken-Ichi
No. 1-67, Futatsuya
Kitamoto-shi Saitama(JP)

(72) Inventor: Miyazaki, Shigeru
No. 1-25, Fujimi-cho
Kohnosu-shi Saitama(JP)

(72) Inventor: Kadota, Shigenobu
No. 1-18-2-608, Higashi juju
Kita-ku Tokyo(JP)

(74) Representative: Geering, Keith Edwin et al,
REDDIE & GROSE 16 Theobalds Road
London WC1X 8PL(GB)

## (54) Nocardia microorganisms and antibiotic product thereof.

(57) A novel antibiotic YS-02931K-β of formula:

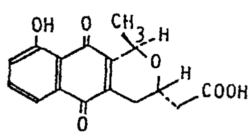

is produced by culturing novel microorganisms of the genus Nocardia and harvesting the resulting antibiotic.

EP 0 186 520 A2

NOCARDIA MICROORGANISMS AND ANTIBIOTIC PRODUCT THEREOF

This invention relates to a new microorganisms of the genus Nocardia, to fermentation of these microorganisms for producing antibiotic YS-02931K-β and to this antibiotic per se. The YS-02931K-β substance obtained by the process of this invention is a compound having high antimicrobial activity against dermatophytes and Gram-positive bacteria.

The YS-02931K-β substance is a compound identifed by the structural formula and physical/chemical properties shown below.

Structural Formula:

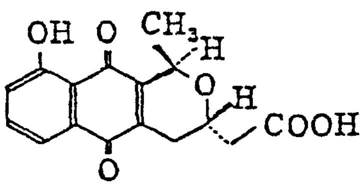

Physical/Chemical Properties:

(1) UV spectrum: The UV spectrum in methanol is shown in Fig. 1.

(2) IR spectrum: The IR spectrum ( KBr disk ) is shown in Fig. 2.

(3) NMR spectrum: The NMR spectrum ( 100 MHz; in $CDCl_3$ ) is shown in Fig. 3.

(4) Molecular weight and molecular formula: 302, $C_{16}H_{14}O_6$

(5) Appearance: Yellow powder

(6) Basic, neutral or acidic nature: Acidic substance

(7) Specific rotation: $[\alpha]_D^{25}$ +26° ~ 27° ( c = 1, MeOH )

(8) Solubility: Soluble in methanol, ethanol, acetone, ethyl acetate and chloroform.

(9) Color reaction: Ferric chloride Positive

Ninhydrin Negative

Dragendorff Negative

(10) $R_f$ value in thin layer chromatography: Silica gel $60F_{254}$ ( Merck ) used; detected with 254 nm UV

| Developing solution | $R_f$ value |
|---|---|
| Chloroform:Methanol ( 3:1 ) | 0.57 |
| Chloroform:Methanol ( 7:1 ) | 0.34 |

As a known compound related to YS-02931K-β substance, there may be mentioned OS-3966-A ( German Patent No.2510868) represented by the following planar structural formula :

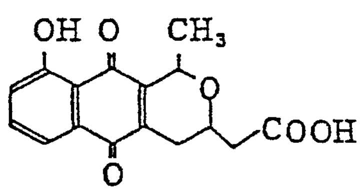

OS—3966—A

According to the description in that patent, OS-3966-A is a substance collected from a culture medium in which a microorganism belonging to the genus Streptomyces has been grown under aerobic conditions and which shows a specific rotation of $[\alpha]_D^{26}$ $-27.5^{\circ}$ ( c = 1.0, MeOH ). The inventors of that patent later named this compound Nanaomycin-A and gave the following steric configuration:

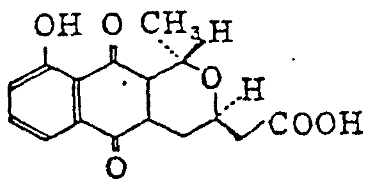

Nanaomycin-A

(cf. J. Chemical Society, Chem. Comm. (1976) 9, 320-321

Therefore the YS-02931K-$\beta$ substance produced by the process of this invention is a compound which is different from OS-3966-A or Nanaomycin-A in steric configuration.

The YS-02931K-$\beta$ substance exhibits high antimicrobial activity against Gram-positive bacteria, yeast and true fungi (eumycetes), and is effective for the prevention and treatment of infections caused by these microorganisms. Activities of YS-02931K-$\beta$ substance against typical microorganisms are listed below in comparison with those of Nanaomycin-A.

Measurement of antimicrobial activity:

To the antibiotic substance was added methanol to make a 250 $\gamma$/ml solution. Thin paper disks for antimicrobial activity measurement ( 8 mm in diameter; Toyo Seisakusho ) were soaked with this solution, excess liquid was removed, and the disks were dried. Using each disk thus prepared, paper disk assay was performed for various authorized microorganisms; growth-inhibition circle diameter was measured after 16 hours' of culture ( at 37°C ) for bacteria and after 48 hours' of culture ( at 27° ) for true fungi (eumycetes).

| Microorganism Tested | Inhibition Circle ( mm ) | Nanaomycin-A | Medium |
|---|---|---|---|
| Baccilus subtilis ATCC 6633 | 21.0 | 14.5 | A |
| Staphylococcus aureus 209P | 23.5 | 21.5 | A |
| Staphylococcus epidermidis S-998 | 26.0 | 17.0 | B |
| Streptococcus faecalis CAY 04/1 | 15.0 | 13.0 | B |
| Candida albicans ATCC 10231 | 13.0 | ( 13.0 ) | C |
| Tricophyton mentagrophytes | 36.5 | 34.0 | D |
| Tricophyton interdigitale | 28.0 | 31.0 | C |
| Penicillium citrirum ATCC 9849 | 23.5 | 19.5 | D |

A: Muller-Hinton's agar medium ( product of Eiken )

B: Hart infusion agar medium ( Product of Eiken )

C: Sabouraud's agar medium ( product of Eiken )

D: Potato-dextrose agar medium ( product of Eiken )

( ): Semi-inhibition

YS-02931K-β substance, which differs from Nanaomycin-A in steric configuration, can be selectively obtained by growing novel microorganisms according to the invention which belong to the genus Nocardia and are capable of producing said YS-02931K-β substance.

The microorganisms according to the invention, including new species Nocardia sp. YS-02931, are collected from soil. An example of the new species of microorganism according to the invention is Nocardia sp.YS-02931K, which is a type strain of the species.

The species and strain designations YS-02931 and YS-02931K used herein are our temporary informal nomenclature pending allocation of formal permant names.

Bacteriological properties of the type strain Nocardia sp. YS-02931K, are described below.

1. Morphology

Nocardia sp. YS-02931K strain forms no aerial mycelium when grown on organic or inorganic agar media. When grown on a favorable medium, its basal hyphae are comparatively long and grow straight with irregular branching, but sometimes extend in a zig-zag way.

Round protrusions like wens are often observed at the top of basal hyphae, indicating their growth by acropetal budding. Spores are elliptical in shape and have smooth surfaces, each measuring 0.8 ~ 1.2 x 2.0 ~ 3.5 mm, and part of hyphae tend to break in the later stage of culture, showing a bacillary shape. Presence of verticillate spores, sclerotium-like structure, sporangia and zoospores was not detectd. The species is negative to acid-fast staining.

2. Behavior on Various Agar Media

The behavior of YS-02931K strain on various agar media is enumerated below. Unless otherwise specified, cultivation was performed at 28°C for 21 days and observation was in the usual way. Color was represented in accordance with the color standard ( Japan Color Research Institute ).

| | | |
|---|---|---|
| Glucose/asparagine agar medium | G | Moderate |
| | A | No deposition |
| | R | Light yellow-brown ~ yellow-brown |
| | S | None |
| Glycerol/asparagine agar medium ( ISP-5 ) | G | Moderate; light yellow-brown |
| | A | No depositioin |
| | R | Brownish white ~ light yellow-brown |
| | S | None |
| Starch/inorganic salt agar medium ( ISP-4 ) | G | Favorable; light yellow-brown |
| | A | No deposition |
| | R | Colorless ~ light yellow--brown |
| | S | None |
| Tyrosine agar medium ( ISP-7 ) | G | Favorable; grayish yellow-brown |
| | A | No deposition |
| | R | Light yellow-brown ~ yellow brown |
| | S | Brown |
| Nutritional agar medium | G | Moderate; yellow-brown |
| | A | No deposition |
| | R | Light yellow-brown ~ yellow brown |
| | S | None |
| Yeast/malt agar medium ( ISP-2 ) | G | Moderate; yellow-brown |
| | A | No deposition |
| | R | Colorless light yellow- |

| | | |
|---|---|---|
| | | brown ～ yellow-brown |
| | S | None |
| Oatmeal agar medium ( ISP-3 ) | G | Moderate; light yellow-brown |
| | A | No deposition |
| | R | Light yellow-brown ～ yellow brown |
| | S | None |
| Bennett's agar medium | G | Moderate; yellow-brown |
| | A | No deposition |
| | R | Light yellow-brown ～ yellow-brown |
| | S | None |
| Peptone/yeast/iron agar medium ( ISP-6 ) | G | Moderate; light yellow-brown |
| | A | No deposition |
| | R | Light yelow-brown ～ yellow brown |
| | S | None |

( Note ) G: Condition of growth; color of colony surface
A: Deposition of aerial mycelium
R: Color of back surface; S: Soluble pigment

3. Physiological Properties

| Items | Observations |
|---|---|
| 1) Growth temperature range | 25 ～ 45°C |
| Optimal growth temperature range | 33 ～ 37°C |
| 2) Liquefaction of gelatin | |
| Gelatin alone ( 20°C ) | Positive |
| Glucose/peptone/gelatin ( 28°C ) | Positive |

| | | |
|---|---|---|
| 3) | Coagulation of nonfat milk | Negative |
| | Peptonization of nonfat milk | Positive |
| 4) | Reduction of nitrates | Negative |
| 5) | Hydrolysis of starch | Positive |
| 6) | Formation of melanin-like pigment | |
| | Tyrosine agar | Positive |
| | Peptone/yeast/iron agar | Positive |

( Note ) Growth temperature: 7 to 21 days' culture at 5, 10, 15, 20, 25, 28, 30, 33, 37, 40, 45 and 50°C; Action upon milk: at 37°C for 3 to 21 days; The other items: at 28°C for two weeks unless otherwise specified.

4. Assimilation of Carbon Sources

( Pridham/Godlieb agar medium; incubated at 28°C )

| | |
|---|---|
| L-Arabinose | - |
| D-Xylose | - |
| D-Glucose | + |
| D-Fructose | - |
| Sucrose | + |
| Inositol | - |
| L-Rhamnose | - |
| Raffinose | - |
| D-mannitol | ++ |
| Starch | + |

++: Grows favorably

+: Grows normally

±: Grows slightly

-: Fails to grow

5. Analysis of Cell Wall Composition

The cell wall composition of Nocardia sp. YS-02931K and the acid hydrolyzate of the whole cell body were analyzed according to the method of Lechevallier et al. ( Lechevallier, Mp. et al.: PP227-238 in Dietz, A et al. ed., Actinomycetes Taxonomy. SIM special publication No.6, 1980 ). Presence of meso-diaminopimelic acid and galactose was proved, but arabinose, which is characteristic of type IV cell walls, was detected only in a very small amount.

To sum up, YS-02931K strain does not form aerial mycelium at all when grown on various media; its basal hyphae generally grow straight but sometimes extend in a zig-zag way, with protrusions like wens often observed at its top, indicating their growth by acropetal budding; spores are elliptical in shape and are formed basipetally in positions slightly apart from the top of hyphae; part of hyphae tend to break in the later stage of culture, but only to a limited extent. The analytical result on the compositions of cell walls and sugars involved suggests that this microorganism falls in the category of type IV cell wall ( though only little arabinose was detected ). No sporangium or mobile spore was detected.

Judging from the morphological properties and cell wall type, species YS-02931 might belong to either genus Nocardia or Pseudonocardia. It is similar to the species of Pseudonocardia in that its hyphae grow by acropetal budding, its spores are elliptical in shape, and its basal hyphae

sometimes extend in a zig-zag way. However, it forms no aerial mycelium, while the species of Pseudonocardia generally show deposition of white mycelia, in powdery form or in thick layers, on the basal hyphae.

In addition, the species of Pseudonocardia have no ability to decompose starch, or to peptonize or coagulate nonfat milk, unlike YS-02931. Taking these facts into consideration, it seems reasonable to consider that the new microorganisms belong to the morphological group III of Nocardia as specified in Bergey's Manual of Determinative Bacteriology, 8th edition (1974). Since they are not identical to any species in this group, we identified them as a new species of Nocardia and named it Nocardia sp. YS-02931, and the type strain YS-02931K has been deposited at the Fermentation Research Institute under Agency of Industrial Science and Technology as FERM-PNo.7962. Clearly, other microorganisms of genus Nocardia having the characteristic bacteriological properties of strain YS-02931K belong to the novel species designated YS-02931.

Microorganisms are generally subject to natural and artificial mutation; the invention includes strains isolated from the natural environment, strains derived by artificial variation e.g. by the action of UV rays, X-rays or chemical agents, and natural mutants (e.g. natural and artificial

mutants and derivatives of type strain YS-12931K).

YS-02931K-β substance can be obtained by growing Nocardia YS-02931 in a suitable medium. Any commonly used culture method may be employed, but submerged culture in a liquid medium is normally preferable.

Any culture media containing nutrients assimilable by Nocardia YS-02931 may serve the purpose. These include synthetic, semi-synthetic and natural media, which contain glucose, arabinose, fructose, starch, vegetable oil, etc., as carbon source; and meat extract, peptone, gluten meal, cottonseed-oil cake, soybean meal, peanut meal, fish meal, corn steep liquor, dry yeast, yeast extract, ammonium sulfate, ammonium nitrate, urea, etc., as nitrogen source. Salts of Na, K, Mg, Ca, Zn, Fe and other metals ( sulfates, nitrates, chlorides, carbonates, phosphates, etc. ) may also be contained as required.

These culture media may also include, as necessary, an accelerator for antibiotic formation or a defoamer, such as methionine, cysteine, cystine, thiosulfates, methyl oleate, lard oil, silicone oil and surface-active agents.

Fermentation under aerobic conditions is generally advantageous, and should preferably be carried out at about 18 to 35°C ( most preferably at about 30°C ). pH of the

medium is best held in the range from about 5 to 10, more preferably from about 5 to 7, to obtain satisfactory results.

YS-02931K-β substance can be isolated by known methods commonly used for isolation of antibiotics from microbial cultures. This substance, produced principally in the culture liquid, is isolated by first removing the microbial cells by centrifugation or filtration, followed by extraction of the effective compound from the supernatant or filtrate. Isolation and purification can be effected by known techniques commonly employed for the manufacture of antibiotics ( those utilizing difference in various properties, such as solubility, precipitation, adsorption and distribution between two liquid phases ), which may be used alone, in any desired combinations, or repeatedly.

The invention is further illustrated by the following example.

Example

Culture medium ( pH: 7.1 ) containing 3.0% potato starch, 1.5% soybean meal, 0.5% corn steep liquor, 0.2% yeast extract, 0.05% magnesium sulfate hepta hydrate, 0.3% sodium chloride and 0.002% cobalt chloride hexahydrate was prepared. This medium was dispensed into 500ml conical flasks ( 60 ml in each ) and sterilized at 120°C for 20

minutes. A pinch of hyphae of Nocardia sp. YS-02931K strain grown on Bennett's agar medium was added to the sterile medium prepared above, and subjected to shake culture at 27°C for 72 hours to make a master culture. Twenty-five liters of culture medium of the same composition as above and also containing 0.03% Adekanol ( product of Asahi Denka Kogyo ) was placed in a 30-liter stainless steel fermentor, and the master culture obtained above was charged in an amount of 3.0%. Fermentation was continued at 28.0-28.5°C for 72 hours ( aeration: 20 l/min; agitation: 95-150 rpm ), when the maximum activity against Bacillus subtilis ATCC 6633 strain was achieved. Radiolite #600 ( product of Showa Chemical Industry ) was added to the culture liquid, and the mixture was stirred well and filtered, giving 20 liters of filtrate. It was adjusted to pH 3 by addition of 0.1N-HCl, 20 liters of ethyl acetate was added, the mixture was thoroughly agitated, and the organic layer was collected. The ethyl acetate solution thus obtained was treated with 3 liters of 1%-$NaHCO_3$ solution, and the aqueous layer was collected and acidified to pH 3 with 0.1N-HCl. Three liters of ethyl acetate was added, the mixture was stirred well, and the organic layer was collected and dried over anhydrous sodium sulfate. After filtering off the drying agent, the filtrate was concentrated under reduced

pressure, leaving about 1 g of orange oil. It was dissolved in a small amount of chloroform, the chloroform solution was adsorbed on 50 g of Wakogel C-200 ( Wako Junyaku ) packed in a column, and the adsorbed matters were chromatographed using chloroform:methanol ( 100:1 ) as developing solution. The fractions showing activity against Bacillus subtilis ATCC 6633 were collected and concentrated under reduced presure, giving 70 mg of yellow oil. It was dissolved in a small quantity of methanol, the methanol solution was applied to a silica gel $60F_{254}$ thin-layer plate ( Merck ) along its edge, and thin layer chromatography was performed using chloroform:methanol ( 7:1 ) as developing solution. The portion which showed an $R_f$ value of 0.34 and exhibited activity against Bacillus subtilis ATCC 6633 was scraped off, the collected silica gel powder was treated with chloroform:methanol ( 7:1 ), and the eluate thus obtained was concentrated under reduced presure, affording 4 mg of pure YS-02931K-$\beta$ substance as yellow powder.

As indicated above, in the accompanying drawings

Figure 1 shows the ultraviolet absorption spectrum of YS-02931K-$\beta$ substance, Figure 2 its infrared absorption spectrum, and Figure 3 its NMR spectrum.

C L A I M S :

1. Microorganisms of the genus Nocardia capable of producing antibiotic YS-02931K-β of the formula :

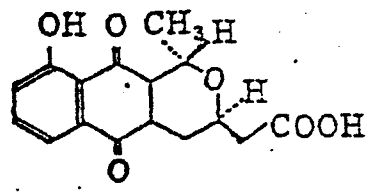

2. The microorganism species YS-02931 of the genus Nocardia capable of producing antibiotic YS-02931K- β of the formula:

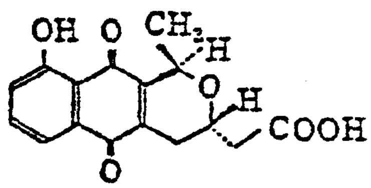

3. A microorganism of the species according to claim 2 which is the type strain YS-02931K.

4. A microorganism according to claim 1 which is a mutant or derivative of the type strain according to claim 3.

5. Antibiotic YS-02931K- β of the formula:

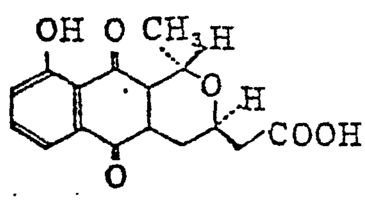

6. A method of making antibiotic YS-02931K-β of the formula :

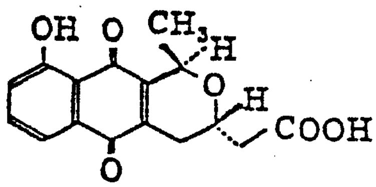

which comprises culturing microorganisms according to any of claims 1 to 4 and harvesting the resulting antibiotic.

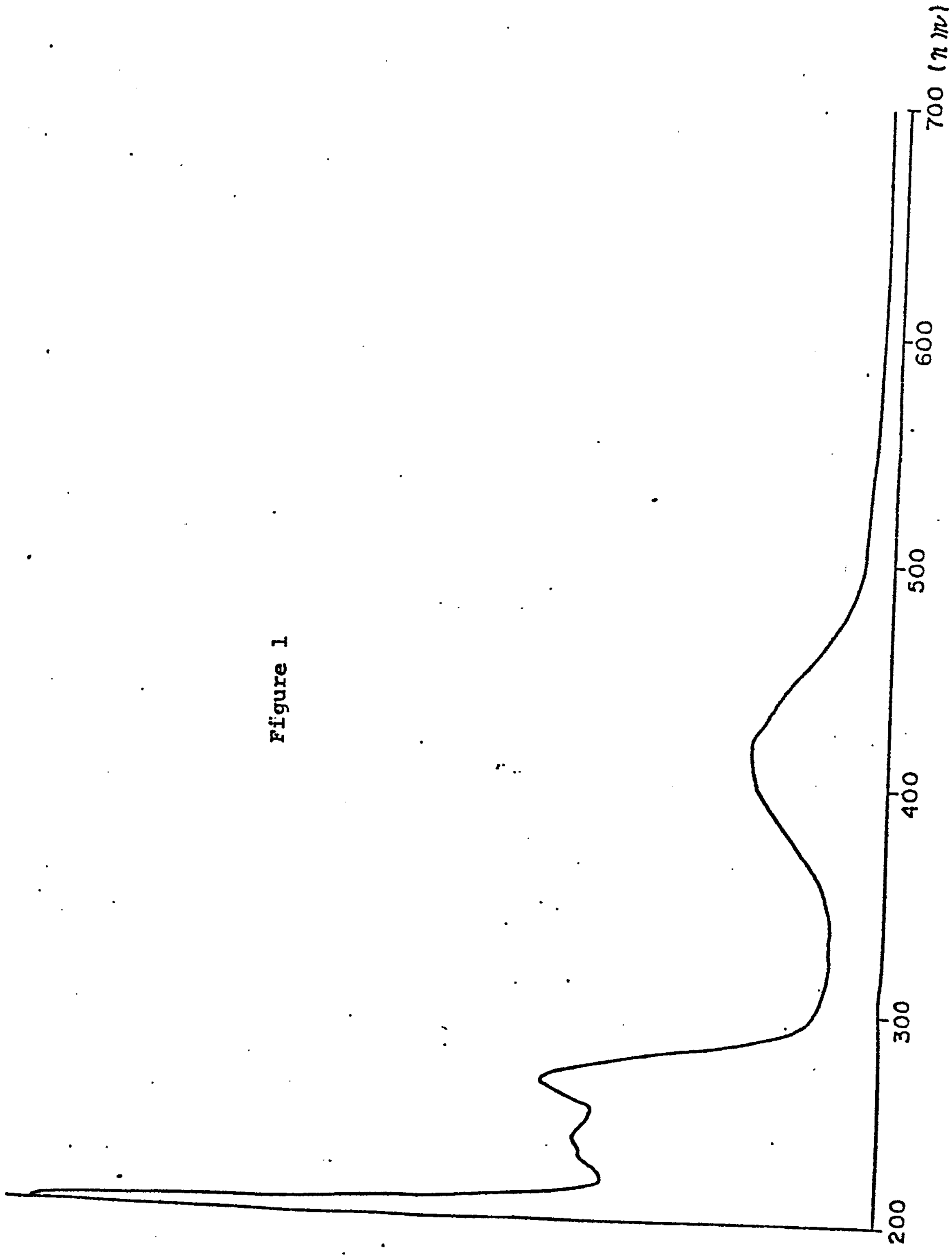


Figure 1

Figure 2

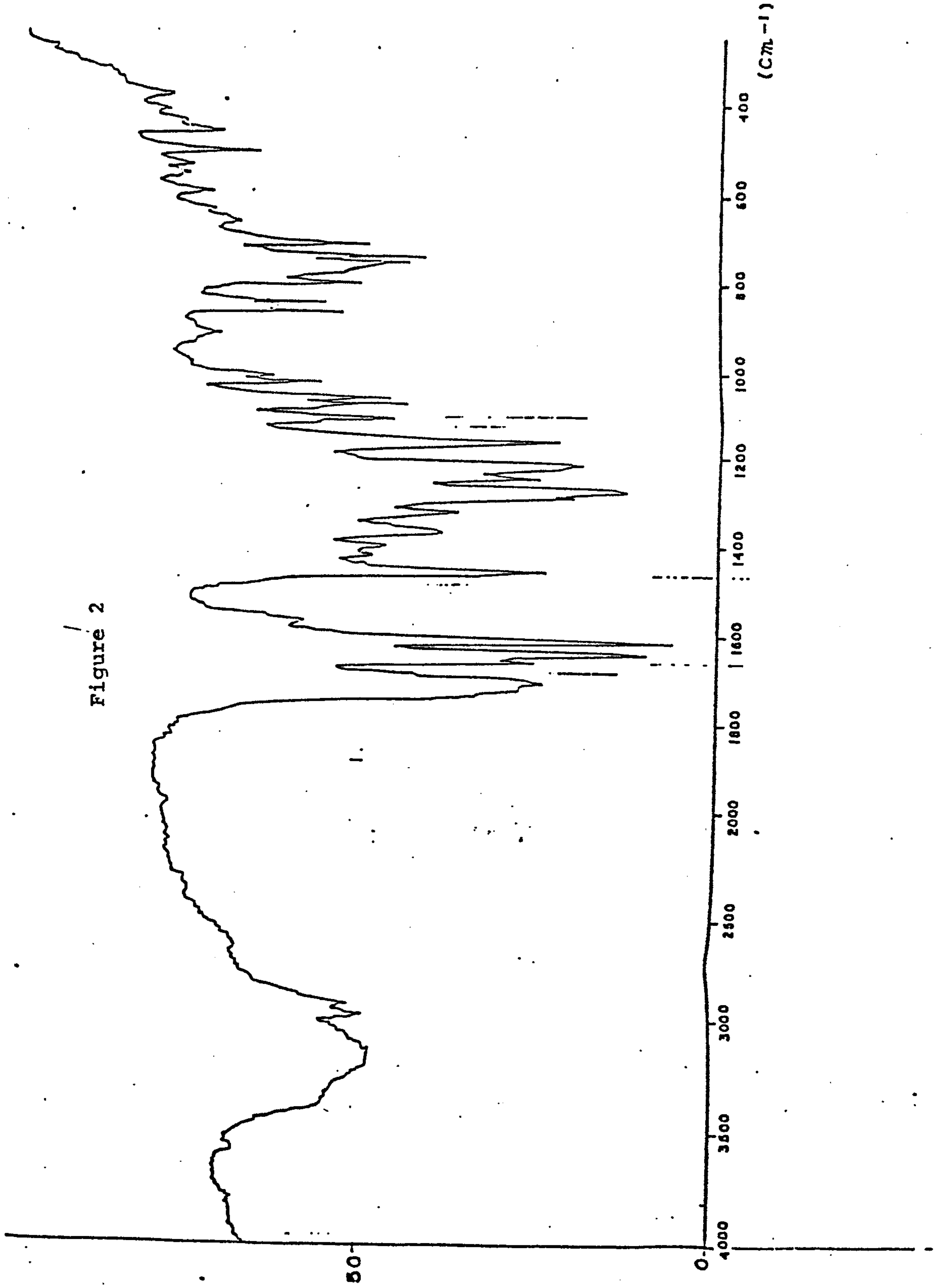
(cm-1)
400
600
800
1000
1200
1400
1600
1800
2000
2500
3000
3500
4000
50
0

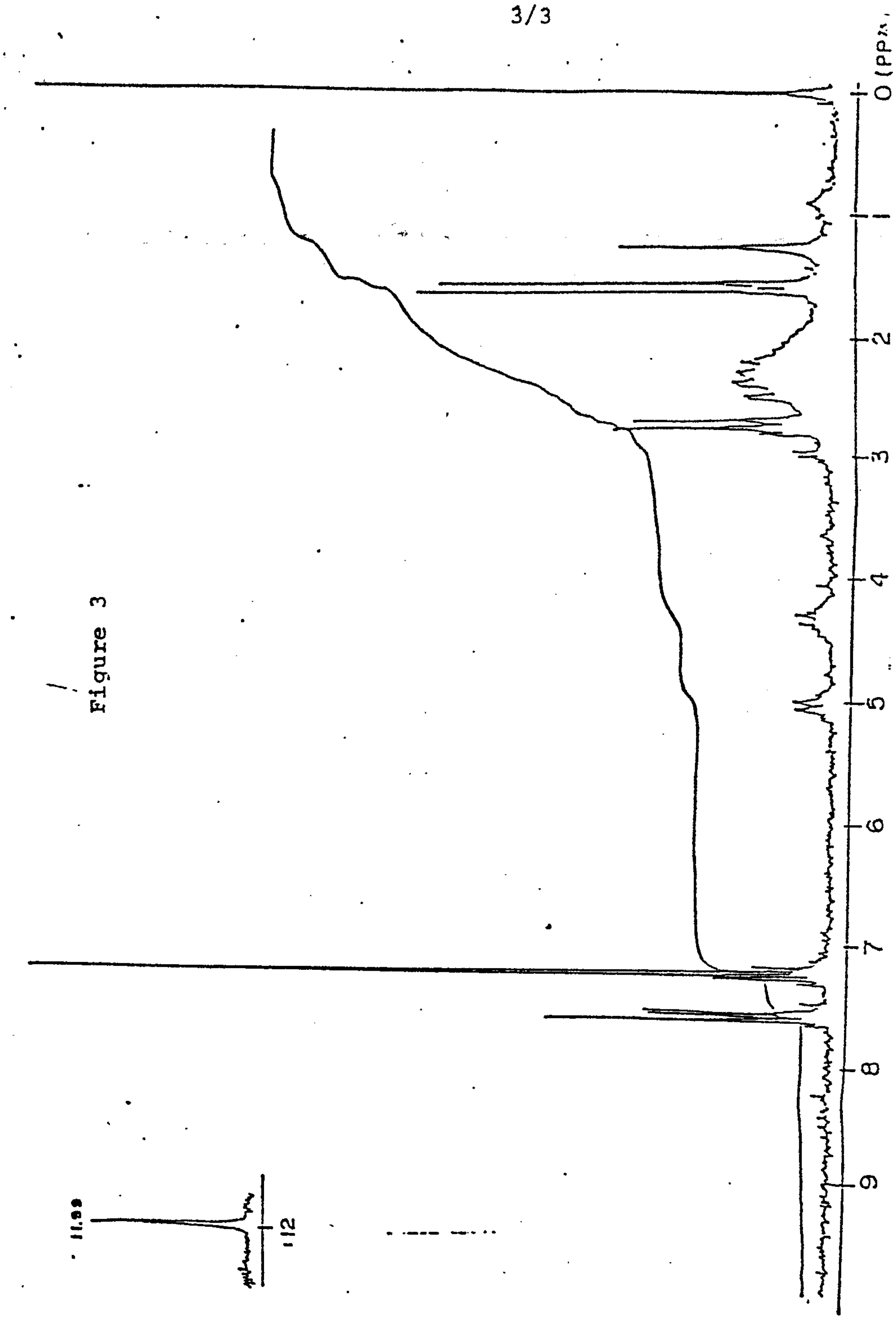


Figure 3